# EUROPEAN PATENT APPLICATION

(11) **EP 4 394 793 A1**
(43) Date of publication of application: **03.07.2024**
(21) Application number: 23218677.5
(22) Date of filing: 20.12.2023
(51) Int. Cl.: G16H 40/20, G16H 50/20, G16H 50/70, G16H 40/67, G06Q 10/00

(54) **PATIENT TRANSFER RECOMMENDATION RESPONSIVE TO EVENT IDENTIFICATION**

(30) Priority: 31.12.2022 US 202263478129 P; 01.02.2023 US 202318104417
(71) Applicant: TeleTracking Technologies, Inc., Pittsburgh, PA 15222 (US)
(72) Inventor: JUBECK, Scott, Pittsburgh, Pennsylvania 15222 (US); COEN, Michael, Pittsburgh, Pennsylvania 15222 (US); IASELLA, Jeanne, Pittsburgh, Pennsylvania 15222 (US); HAVRANEK, Thomas, Pittsburgh, Pennsylvania 15222 (US)
(74) Representative: Sandri, Sandro

(57) **Abstract**

One embodiment provides a method, the method including: identifying, at an event response recommendation system, an event affecting a healthcare facility; identifying, using the event response recommendation system, at least one patient within the healthcare facility needing transferred due to the event; and generating, using the event response recommendation system, a recommendation for transferring the at least one patient. Other aspects are described and claimed.

## Description

### BACKGROUND

When an event (e.g., wildfire, cyberattack, tsunami, earthquake, hurricane, etc.) strikes, healthcare facilities may be uniquely affected. Since healthcare facilities provide healthcare and healthcare services to entire communities, regions, and/or the like, when an event strikes, more than just a single household is affected. One way to support a healthcare facility in the face of an impending event or even once an event has affected a healthcare facility, is to evacuate patients. This is particularly true if the healthcare facility is expected to not be able to or can no longer appropriately care for the patients. Another reason that patient transfers may be useful when an event affects and area is to get more stable patients to other facilities, thereby opening up a healthcare facility within the affected area to treat new patients, particularly emergent or critical need patients.

### BRIEF SUMMARY

In summary, one aspect provides a method including the steps described at claim 1. The dependent claims outline advantageous ways of carrying out the method.

Another aspect provides a system including: a processor; a memory device that stores instructions that, when executed by the processor, causes the system to carry out a method according to any one of the preceding claims.

A further aspect provides a computer program product including: a computer-readable storage device that stores executable code that, when executed by a processor, causes the product to: identify, at an event response recommendation system, an event affecting a healthcare facility; identify, using the event response recommendation system, at least one patient within the healthcare facility needing transferred due to the event; and generate, using the event response recommendation system, a recommendation for transferring the at least one patient.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates an example method of generating a recommendation for transferring a patient from a healthcare facility responsive to an event affecting the healthcare facility.
FIG. 2 illustrates an example of device circuitry.

### DETAILED DESCRIPTION

In the face of an impending event or once an event has affected a healthcare facility, evacuations of patients within the healthcare facility may be performed. Since healthcare facilities can have different level of treatment capabilities, the patient has to be transferred to a healthcare facility that has at least the same level of treatment capabilities as needed by the patient. Additionally, the patient needs to be transferred to a facility that also will not be affected by the event. Finally, the healthcare facility that may be receiving the patient needs to not only have a bed for the patient, but also must have the same level of care available to the patient. For example, if the patient is in need of acute medical care, the patient must be transferred to a healthcare facility that not only provides acute medical care, but also has an available bed within the acute medical care department.

Conventionally, transferring patients is a manual process where staff of the healthcare facility needing to transfer a patient must communicate with other healthcare facilities to identify availabilities. Once a healthcare facility is identified, the staff must make arrangements for the transfer of the patient to the healthcare facility. While this may be an inconvenience for a single patient, it becomes a very time-consuming laborious process when many patients are involved, for example, an entire healthcare facility. Additionally, it is unlikely that a single healthcare facility will be able to take all patients that need transferred, so the healthcare facility has to perform this same process with multiple healthcare facilities to ensure that all patients can be transferred. This process can become very lengthy which can become a particular process in view of a looming event. Additionally, if an event has already occurred, patients may need to be immediately transferred with time being of the essence. The slow manual conventional process of finding a healthcare facility that can take the patient and actually performing the transfer can become a significant problem in these cases.

Accordingly, the described system and method provides a system and method for generating a recommendation for transferring a patient from a healthcare facility responsive to an event affecting the healthcare facility. An event response recommendation system is employed that identifies an event affecting a healthcare facility. In view of the event, the event response recommendation system identifies at least one patient that needs to be transferred from the healthcare facility. The transfer is generally needed because the healthcare facility is being evacuated in view of the event. The evacuation may be a full healthcare facility evacuation or may only be a partial evacuation with certain patients, departments, and/or the like being evacuated.

In addition to identifying the patient(s) that need to be transferred, the event response recommendation system may identify characteristics of the patient(s). These characteristics may be health characteristics that may influence a healthcare facility to which the patient could be transferred. Some example characteristics include a care level of the patient, special accommodations for the patient, a preference of the patient, healthcare objects required for the patient, and/or the like. After identifying the at least one patient, the event response recommendation system generates a recommendation for transferring the at least one patient. The recommendation includes identifying a healthcare facility to which the patient can be transferred. When making the recommendation, the event response recommendation system may take into account the patient characteristics, meaning the system attempts to generate a recommendation that fulfills the patient characteristics. The system may also attempt to optimize the recommendations in view of all the patients that need transferred from the healthcare facility.

Once the recommendation has been generated, the event response recommendation system may perform one or more actions. One action may include presenting the recommendation(s) to a user for review and/or approval. The event response recommendation system may also initiate the transfer for the patient(s) based upon the recommendation. In the event that the recommendation(s) were provided to a user, the transfer initiation may be responsive to the user approving the recommendation. In initiating the transfer, the event response recommendation system may generate transfer order, initiate transportation requests, order healthcare supplies, and/or the like, to facilitate the transfer. The system may also track the orders and effectuate the performance of the orders. Once the patient is transferred to another healthcare facility, the event response recommendation system may track the patient and provide notifications and alerts the transferring healthcare facility about a status of the patient. Additionally, in the event the healthcare facility becomes operable or able to care for the patient again, the event response recommendation system may initiate a transfer of the patient back to the transferring healthcare facility.

The described system and method will be discussed with respect to a healthcare system or healthcare facility for ease of readability. Additionally, for simplicity, the term "hospital" may be used here throughout. However, it should be understood by one skilled in the art that this term may refer to any healthcare system or facility, for example, long-term care facility, emergency department, healthcare staffing area, hospital facility including multiple departments within a healthcare campus or affiliations, affiliated or otherwise connected healthcare systems, and the like. However, the fact that this disclosure is discussed with respect to a healthcare system or hospital is not intended to limit this disclosure to only scheduling within a healthcare system. As can be understood by one skilled in the art, the disclosed systems and methods can be applied to any entity that transfers people or objects between facilities.

Additionally, while the disclosure refers to an event affecting a healthcare facility, it should be noted that an event may only affect a portion of the healthcare facility or may affect the entire facility. For example, the operating room department may be closed, but the intensive care unit may remain open. As another example, the event may cause the entire facility to need to be shut down.

The illustrated example embodiments will be best understood by reference to the figures. The following description is intended only by way of example, and simply illustrates certain example embodiments.

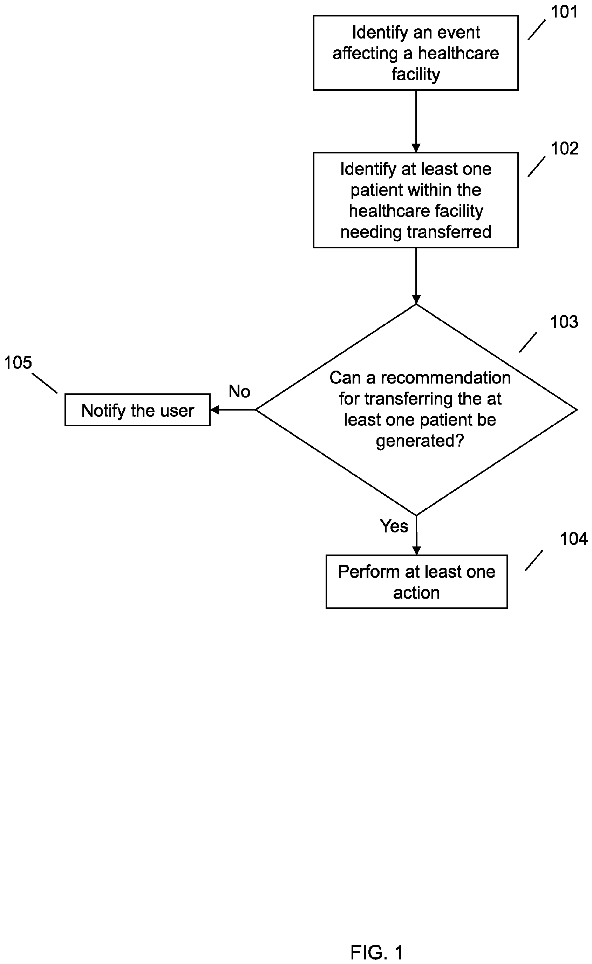

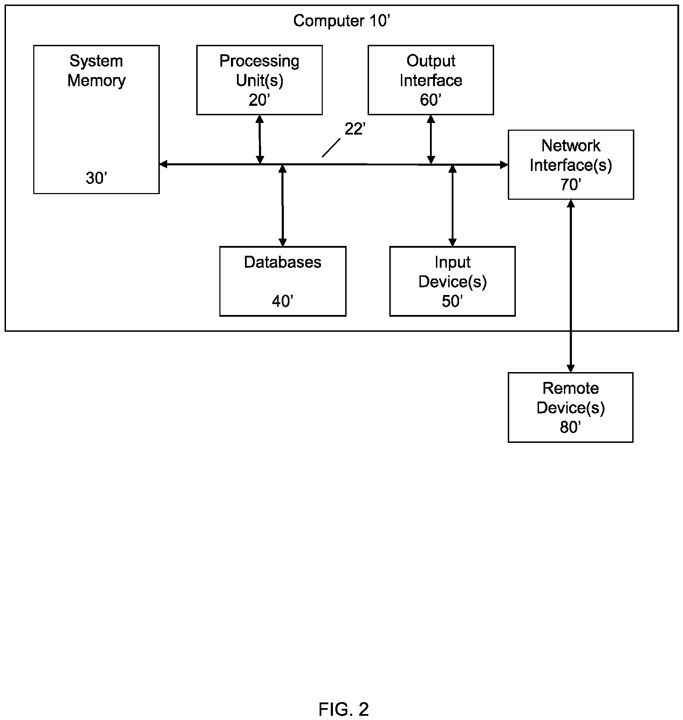

FIG. 1 illustrates a method for generating a recommendation for transferring a patient from a healthcare facility responsive to an event affecting the healthcare facility. The method may be implemented on a system which includes a processor, memory device, output devices (e.g., display device, printer, etc.), input devices (e.g., keyboard, touch screen, mouse, microphones, sensors, biometric scanners, etc.), image capture devices, and/or other components, for example, those discussed in connection with FIG. 2. While the system may include known hardware and software components and/or hardware and software components developed in the future, the system itself is specifically programmed to perform the functions as described herein to generate recommendations for patient transfers. Additionally, the event response recommendation system includes modules and features that are unique to the described system.

The event response recommendation system provides a software application that may include a graphical user interface, as described in more detail below. While the software application does provide a graphical user interface, the software application also provides backend functions and other functions other than the graphical user interface. These will be discussed in more detail further herein. Thus, the event response recommendation system may provide a software application and, within the software application, may provide a graphical user interface that allows for receiving user input.

Once the user input is received, the event response recommendation system can analyze the user input to perform the steps as further described herein, for example, identifying an event affecting a healthcare facility, identifying at least one patient within the healthcare facility that needs to be transferred due to the event, and generate a recommendation for transferring the patient. The system may also perform additional functions, for example, automatically initiating a transfer for a patient, which may include generating transfer orders and effectuating those transfer orders. It should be noted that each of the steps performed by the system may allow for user input through the graphical user interface. Additionally, the system may also update the graphical user interface with a status of a patient, evacuation of the healthcare facility, and/or other statuses that may be generated or monitored.

The event response recommendation system, also referred to also as a recommendation system and system, may include or have access to network devices. The network devices may facilitate communication between the event response recommendation system and another device, component, facility, healthcare system, and/or the like. For example, the recommendation system may utilize a network device to communicate with the healthcare systems applications, programs, and/or systems, that are providing and receiving transmissions.

The event response recommendation system is provided that allows for interface between one and more users and other systems within the healthcare facility and the event response recommendation system and other systems of the hospital or other systems of other healthcare facilities. The recommendation system may communicate and transmit information with other hospital systems that may provide information regarding patients, healthcare professionals, hospital objects, support personnel or departments, and/or the like. For example, the recommendation system may interface with an object tracking system, patient registration system, employee system, support personnel or department system, and/or the like. Thus, the recommendation system can pull information from each of the interconnected or accessible systems to identify patients needing to be transferred in the case of an event, characteristics of those patients, and attributes of other healthcare facilities and generate recommendations for patient transfers. The recommendation system can also transmit information to one or more of the interconnected or accessible systems.

The recommendation system may allow interconnectivity of healthcare facilities and systems across nations and/or globally. These interconnected healthcare facilities and systems may provide information to each other so that each individual healthcare facility may be apprised of a status of a different healthcare facility and/or system in near real-time without having to manually request the information from another healthcare facility. This allows for accurate recommendation generation. This provision of status information from each healthcare facility to another facilitate through the event recommendation system is referred to as a heartbeat. This status information is provided consistently, at a predetermined frequency, so that the most accurate recommendations can be generated.

The recommendation system may be a background application that does not have an associated display or monitor. Rather, the recommendation system, when accessed by a user will provide instructions to generate and display a graphical user interface on a display device utilized by the user. Thus, the recommendation system may not have its own display device, but does have the ability to generate and display a graphical user interface. The graphical user interface is updated and managed based upon instructions provided by the recommendation system. In other words, the recommendation system generates and transmits instructions to create and update the graphical user interface. Alternatively, or additionally, the recommendation system may be a standalone system that has an associated display device. In this case, the recommendation system still provides instructions for generating a graphical user interface as further described herein.

The recommendation system may also have a standalone system and may also be accessible through other computing devices. For example, the recommendation system may be a standalone system that can be accessed by a user and also may be an application that is accessible by a user on another computing device. The recommendation system may be accessible using any type of computing device, for example, personal computer, laptop computer, smartphone, tablet, smartwatch, head-mounted display, smart television or other smart appliance, and/or the like.

The graphical user interface may provide a user with the ability to access the recommendations generated by the recommendation system. Thus, the graphical user interface displays a generated recommendation, a progress of generating a recommendation, errors or alerts associated with generating a recommendation, a status of a transfer initiated by the recommendation system, and/or the like. The recommendation and information associated with the recommendation may be the default view when the graphical user interface is first accessed. The default view may also be a user input screen that allows the user to provide input to identify a particular event, a particular patient, a particular transfer, and/or the like, that the user wants to view. Alternatively, or as configured by the system or user, the recommendation and associated information may be displayed when a user interfaces with a recommendation icon. Since multiple recommendations may be made, the graphical user interface may provide the ability to access a single recommendation, for example, by selecting a particular patient, or a group of recommendations, for example, those recommendations for a particular destination healthcare facility, those recommendations for all patients being transferred at a particular time, those recommendations for all patients being transferred due to a particular event, and/or the like.

It should be noted that different users may configure the graphical user interface per their preferences. Thus, the graphical user interface layout and configuration may be different between users. How much a user can configure the layout may be restricted or set by a system administrator and/or the like. Additionally, different users or different user roles may have different levels of access, which may also change how and what information is displayed. Thus, different graphical user interfaces may be displayed by the system.

The graphical user interface may also provide different icons that allow a user to configure the system, including setting user or system preferences or defaults, identifying automatic notification recipients, identifying users having access to different portions of the recommendation system, providing interface information to interface with different hospital systems, facilitating updates of the system, and/or any number of other configuration options. The graphical user interface may also provide different icons that allow the user to provide input to the recommendation system, for example, identifying an event; viewing, approving, and/or modifying a generated recommendation; viewing historical information; manipulating views provided to users; and/or any number of other input options.

The graphical user interface may include one or more graphical elements. The graphical elements may include user input fields that facilitate the generation of a recommendation. The user input fields may also allow a user to provide input to view pending recommendations, historical recommendations, statistics of recommendations (e.g., a frequency of transferring to a particular healthcare facility, an average number of patients that are transferred in view of a particular event, an average length of time to effectuate a transfer, a number of departments involved in a particular or group of transfers, etc.), and/or the like. Input fields may include radial selector buttons, pull-down menus, free-form fields, structured input fields, selection boxes, and/or any other type of input field. The graphical elements may include icons that a user can interact with.

Some icons may also be display icons that display information. The display icons may be static icons that have static visual elements. The display icons may be dynamic icons that have dynamic visual elements that change based upon an update in information displayed on the icon, periodically, based upon a condition being met, and/or the like. For example, the graphical element may be a display icon that displays the status of a recommendation generation and/or a patient transfer. As the status is updated, the graphical element is updated to reflect the new status. In other words, the graphical element is iteratively updated based upon changes to the status of the recommendation generation and/or patient transfer. As should be understood, there may be other dynamic graphical elements, and this is only an example of one. For example, other dynamic graphical elements may include other status indicators, statistic indicators, and/or the like.

Machine-learning models may be used by the event response recommendation system. A machine-learning model, which may be a neural network, decision tree and/or forest, classifiers, random tree forest or classifier, a combination thereof, a combination of machine-learning models, and/or the like, may be utilized in performing one or more acts of the described system. For example, one or more machine-learning models can be used to identify events affecting a healthcare facility, identifying patients needing transferred in view of an event, identifying characteristics of patients needing transferred, generating and optimizing recommendations for transferring a patient, and actions taken by the system after a recommendation is generated, and/or the like. It should be understood that while the terminology may refer to a single machine-learning model, multiple machine-learning models can be utilized in performing one or more functions of the recommendation system. The machine-learning model may include a plurality of layers, including input, output, hidden, a combination thereof, and/or the like, layers. The machine-learning model is very complex and utilizes complicated mathematical computations. Due to the complexity of the machine-learning model, it would be impossible to perform the analysis as performed by the model in the human mind.

Additionally, the machine-learning model is trained to or utilized to make predictions on data that has been previously unseen by the model. To make these predictions, the model includes very complicated mathematical computations that would not be performed in the human mind. Rather, the use of a computer and processor, and, possibly a computer and processor that is specific and tuned to the machine-learning model, allows for performing these complex computations, particularly with a speed that allows for performing the complex processing found in and required by the machine-learning model in a time frame that facilitates the use of the machine-learning model for making the predictions. This speed is not possible with a human or even a group of humans. Thus, a human or even a group of humans, even using pen and paper, could not perform the analysis performed by the machine-learning model in a manner that would actually result in making the predictions provided by the machine-learning model on the large amount of data that is received by the recommendation system in a length of time that would make the recommendation system function as intended.

The machine-learning model may be trained using a training dataset having annotated training data. Annotated training data includes data that the model may make a prediction upon where the data is annotated with the correct prediction. The machine-learning model can learn from the training dataset how data should be classified or the predictions that should be made with respect to particular data. As predictions are made upon non-annotated data, feedback may be provided. Feedback may be provided in the form of a user making a correction, a user providing input regarding the prediction, predictions from other models regarding the same data, and/or the like. The feedback can be automatically ingested by the model to further train the model, thereby making the model more accurate over time. It should be noted that the model can monitor the predictions made by the model to identify the feedback so that a user does not have manually provide the feedback to the model. Thus, while the model may initially be trained with a training dataset, the model can continually be trained as it is deployed using predictions and feedback regarding the predictions.

In the case of the generation of recommendations, the machine-learning model may be trained using recommendation data and may, therefore, be able to generate recommendations for patients in a healthcare facility. Similar training and corresponding predications may be used for other portions of the described system. The machine-learning model also be trained using unsupervised learning techniques, other supervised learning techniques, reinforcement training, a combination thereof, and/or the like.

At 101, an event response recommendation system identifies an event that is affecting a healthcare facility. It should be noted that the event may be currently affecting the facility or may be an impending event that is expected to affect the healthcare facility. The event data identification and collection system may receive an indication that an event is affecting a healthcare facility from the facility itself, from an entity attempting to support a healthcare facility, from a secondary source (e.g., news, social media site, healthcare facility communication transmissions, etc.), and/or the like. Thus, the event identification may be a manual process and/or an automatic process.

Once an event affecting a healthcare facility has been identified, the event data identification and collection system may receive an indication of the event affecting a healthcare facility. This indication may be a manual indication from a user accessing a graphical user interface of the event response recommendation indicating that an event is affecting or going to affect the healthcare facility. The indication may also be an automated or semi-automated identification of an event affecting the healthcare facility. It should be noted that while the phrase "event affecting the healthcare facility" is utilized, this does not necessarily mean that the event is actively affecting the healthcare facility. Rather, this may be an event that is predicted to affect a facility or may be that the event is actively affecting the healthcare facility.

The event response recommendation system may also identify what type of event is affecting the healthcare facility. Again, this may be a manual process where a user provides an indication of the event type, or may be an automatic process where the system automatically identifies the event type. The event response recommendation system may also identify attributes related to the healthcare facility that is being affected. Different healthcare facilities may have different characteristics or attributes that may provide an indication of a type of healthcare facility that will be needed for transferring the patients to. For example, a geographical location of a healthcare facility may dictate preferred healthcare facilities for transferring patients to. Thus, attributes of the healthcare facility may include a geographical location, resources of the healthcare facility, proximity to other entities, type of healthcare facility (e.g., long-term facility, emergency care facility, short-term recovery facility, general facility, etc.), connection to other healthcare facilities, and/or the like.

As an example, an acute care facility could transfer patients to any type of healthcare facility that can care for acute care facility patients. However, facilities that are not equipped to care for acute care facility patients would be undesirable for acute care facility patients. However, it should be noted that in some cases patients may be transferred to facilities that are not the best suited for the needs of the patient, but this would likely only happen in extreme cases and would not be the preference. Nevertheless, the described system could also identify these cases, provide recommendations accordingly, and initiate those transfers also.

In identifying that an event is affecting a healthcare facility, the system may identify that the facility is operating within an abnormal operating state. An event that may affect a healthcare facility refers to an event that constrains or modifies the normal functioning of the healthcare facility. The possible events may be natural events, for example, earthquakes, tsunamis, wildfires, tornadoes, mudslides, floods, hurricanes, and/or the like. The possible events may be manmade events, for example, cyberattacks, terrorist attacks, construction, conflicts, and/or the like. Other events are also possible, for example, power outages, communication transmission outages, lack of potable water, food or other resource shortages, pandemics, disease transmissions, and/or the like.

Operating normally may be a state that is learned by the system. For example, the system may monitor key datapoints, metrics, dashboards, and/or the like, to determine a normal value or range that those data indicate. Example data that may be monitored and may provide an indication of an abnormal operating state or condition include staffing values, staff-to-patient ratios, patient bed occupancy, resource usage and availability ratios, and/or the like. Values for those data that run within the normal value or range may indicate a normal operating state of the healthcare facility. If the values meet or exceed a predetermined threshold, the system may identify that the facility is no longer operating normally and is, therefore, in an abnormal operating state. To identify an abnormal operating state, the system may require that multiple data values are outside predetermined thresholds, that data values run outside the predetermined thresholds for a predetermined period of time, and/or the like. In addition to learning the normal operating state values, a user may also program the system with some default values and threshold values.

Additionally, the system may monitor certain components or objects within or connected to the healthcare facility and may determine that an outage of one or more of those components or objects indicates an abnormal operating state. For example, the system may monitor power to the healthcare facility, generator functioning, water availability, environmental water levels, weather conditions, and/or the like. The loss of one or more of these components may indicate an abnormal operating state.

To identify a correlation between an abnormal operating state and an event, the system may identify an event that has occurred or is occurring proximal to the identification of the abnormal operating state. Thus, the system may identify a cause of the abnormal operating state and assign that cause as an event. To identify possible causes, the system may monitor one or more information sources that may provide an indication of an event. For example, news, social media sites, Internet searches, healthcare facility communications, and/or the like, may identify an event that has occurred, will be occurring, or is currently occurring that can be correlated to the abnormal operating state. For example, the system may identify that a news report indicated that a wildfire is burning in the area of the healthcare facility. Upon identifying an abnormal operating state, the system may correlate the wildfire as the event affecting the healthcare facility. A user may also manually identify the event causing the abnormal operating state. By identifying the cause of the abnormal operating state, the system has identified an event that is affecting a healthcare facility and can also identify attributes of the event.

Identifying the abnormal operating state can be performed utilizing a machine-learning model, historical information, and/or any other technique. As an example for identifying an event, the system may employ a machine-learning model to identify an event is affecting a healthcare facility and to identify attributes about the event (e.g., event type, event duration, event geographic location, event range, etc.). As another example, the event data identification and collection system may monitor, mine, and analyze secondary sources (e.g., new sources, social media site, Internet searches, healthcare facility communications, etc.) which may be indicative of events affecting healthcare facilities. The system may be able to parse and mine the information to identify events and information related to the events. Depending on the medium of the secondary source, the system may employ one or more parsers (e.g., text parsers, audio parsers, image parsers, etc.), information extraction techniques (e.g., semantic analyzer, information extractors, parts-of-speech analyzers, syntactic analyzers, image analyzers, audio analyzers, etc.), machine-learning models, and/or the like, to analyze and extract information from the secondary sources.

Other techniques for identifying that an event is affecting a healthcare facility are contemplated and possible. For example, when an event affects a healthcare facility, a user within the facility may provide an indication that an event is affecting the healthcare facility. The user may also provide an indication of the type of event that is affecting the healthcare facility. One technique for providing this indication is to access a user interface which may include the graphical user interface discussed further herein. Within the graphical user interface the user may interact with one or more icons or graphical elements to indicate that an event is affecting the healthcare facility and may provide additional information regarding the event and/or affect on the healthcare facility. Another technique for a user providing this indication is a user could update a status of the healthcare facility on an Internet website or other secondary source. The system could then monitor this secondary source and identify that it has been updated to reflect that an event is affecting the healthcare facility.

In addition to identifying that an event is affecting the healthcare facility and identifying a type of the event affecting the healthcare facility, the system may also identify other characteristics regarding the event and/or healthcare facility. Example characteristics of the event other than the event type may include a range of the event, a current duration or expected duration of the event, a starting or expected starting time of the event, a severity of the event, and/or the like. Example characteristics of the healthcare facility include geographical location, proximity to other healthcare facilities, type of healthcare facility, capacity of the healthcare facility, unique characteristics of the facility (e.g., utilizes solar generators vs. fuel generators, has a water purification system vs. not having a water purification system, possible evacuation locations, etc.), and/or the like. The characteristics regarding the event and/or healthcare facility may assist in accurately and effectively providing recommendations for patient transfers in response to an event.

Another technique for identifying an event may be to utilize crowd-sourced data or secondary sources. Many entities have manuals or other documentation that identifies the steps that should be taken when an event occurs at an entity. These events are those that affect the entity. In many cases, these identified events can also affect other entities. Accordingly, the system may access or be provided with entity documentation that includes an identification of events that can affect different entities in a type of crowd-sourcing potential event data. These events can then be identified as events that could affect a healthcare facility.

At 102, the event response recommendation system identifies at least one patient within the healthcare facility needing to be transferred due to the event. In other words, the event response recommendation system identifies patients that need to be moved from the healthcare facility that is affected by the event to another healthcare facility that will not be affected by the event. The patients that need to be transferred may vary based upon the event affecting the healthcare facility. Thus, the number of patients that need to be transferred may be less than the total number of patients that are currently being treated within the healthcare facility. For example, if the event is an event that results in a loss of oxygen provision within the healthcare facility, the patients that need to be transferred are those that are either actively utilizing oxygen or that may need to use oxygen. However, those patients that are not dependent upon and may not become dependent on oxygen may not be transferred. As another example, if the event is one that devastates an area around the healthcare facility, the patients that need to be transferred may be those patients that can be transferred to lower-level care facilities so that any incoming patients can be treated at the affected healthcare facility. As another example, if the event is one that renders the healthcare facility inoperable, the patients that may need to be transferred may be all patients that are being treated in the facility.

In making an identification of the patients that need to be transferred, the system may make a correlation between events and event attributes and patients within the healthcare facility. Event attributes may identify effects that an event may have on a healthcare facility. Example event attributes may include, but are not limited to, event type, event range, event effects, event proximity, event duration, and/or the like. Certain events may have different effects which may be fairly predictable. For example, some events may result in power outages, non-potable water, supply chain delays, evacuation route blockages, oxygen provision reduction or elimination, complete inoperability of the healthcare facility, devastation to an area around the healthcare facility, and/or the like. The events and event effects may be stored in a database of the event response recommendation system and may be accessed by the system when an event is identified. A user may manually upload effects into the database; the system may learn effects from historical information, machine-learning models, crowd-sourced data, and/or the like; download effects into the database; and/or the like.

Once the effects are identified, the system can identify patients that will be affected by these effects. This identification may be made by a correlation between needs of a patient and resources that will be affected by the events. For example, if a patient has a need for oxygen, if an effect of the event is the reduction or elimination of the oxygen, the system may identify that patient as one that needs to be transferred. Similar correlations may be made between other effects and patient needs. The patient needs may be identified based upon the patient characteristics. The correlations may be identified using a rules engine that compares an effect to a rule that identifies a type of patient or patient characteristic that would be affected by that effect. The system can then identify those patients having that type or characteristic. Machine-learning models, crowd-sourcing, historical data analysis, and/or the like, may also be utilized in making these correlations.

In addition to identifying patients that need to be transferred, the event response recommendation system may identify characteristics of the patient(s) needing to be transferred. Characteristics of the patient may include special needs or accommodations for the patient (e.g., wheelchair, oxygen, particular testing requirements, particular monitoring requirements, etc.), a care level of the patient within the healthcare facility (e.g., emergency care, acute care, short-term care, long-term care, hospice, etc.), a preference of the patient (e.g., proximity to a particular location, preferred healthcare facility, preferred healthcare provider, preferred insurance to be taken at a healthcare facility, etc.), insurance of a patient, and/or any other characteristics that may be useful in identifying a healthcare facility the patient can be transferred to. Identifying the characteristics may include accessing a health record of the patient that may include or identify these characteristics or may provide information that allows for these characteristics to be identified.

At 103, the event response recommendation system may determine if a recommendation for transferring the at least one patient be generated. The recommendation may identify a transferee healthcare for transferring the patient. In other words, the recommendation identifies a healthcare facility that can care for the patient and is able to currently take the patient. The event response recommendation system may first identify those healthcare facilities which could take patients that need to be transferred. To make this determination, the system can receive and/or access the status transmission or heartbeat that is received from other healthcare facilities. As previously mentioned, since the healthcare facilities are interconnected, the healthcare facilities transmit information via a heartbeat that identify a status of the healthcare facility. Since the heartbeat is transmitted at a regular, consistent frequency, interconnected healthcare facilities can be apprised of an accurate status of other healthcare facilities.

The heartbeat may contain status information that indicates a capacity of a healthcare facility, a current availability of the healthcare facility for receiving patients, and may also identify what departments or care levels within the healthcare facility have occupancies and how many occupancies within those departments and care levels. Healthcare facilities may also transmit additional information in the heartbeat that may be useful for determining if the healthcare facility would be able to accept patients for transfer. The event response recommendation system may also know or access additional information or attributes regarding a possible healthcare facility to be used for transferring patients. For example, the system may identify a proximity of the potential healthcare facility to the affected healthcare facility, a method of transportation that is available or can be accepted at the potential healthcare facility (e.g., helicopter, ambulance, transport vans, etc.), a preference of the potential healthcare facility by the affected healthcare facility, whether a potential healthcare facility may also be affected by an event, preferences of the potential healthcare facility (e.g., preference for a certain number, type, or care level of patients, insurance preferences, etc.), and/or the like.

Once potential healthcare facilities are identified, the system may attempt to determine which patients should be transferred to which healthcare facilities. The more patients that need to be transferred, the more potential healthcare facilities and attributes of the potential healthcare facilities, and different characteristics may make this a complex algorithm for identifying the healthcare facilities for transferring patients. Within this determination of where to transfer a patient or group of patients to, the system may take into account different factors such as, patient characteristics, priorities of patient characteristics, and preferences of the affected healthcare facility and/or potential healthcare facility. Thus, in making the recommendation the system may attempt to optimize the recommendation and the patient transfers in view of the different factors.

Since the most critical part of transferring a patient is likely to be transferring a patient to a facility that has at least the same level of care that the patient needs and that also has a bed within the needed care level, the system may make a first pass to identify those patients that need to be transferred having the highest needed care level. The system may then identify those healthcare facilities that can meet this needed care level and that also have occupancies within this care level. The system may then identify those healthcare facilities that would be best matches to the patient based upon other factors. Upon a second pass, the system may look at the patients have the next highest care level requirements and identify best matches for those patients. It should be noted that patients can be matched with healthcare facilities having a higher level of care than needed by the patient, due to availability, patient characteristics, and other factors.

This process may continue until all patients have been placed. Additionally, as patients are being placed, the system may identify that a patient could be transferred to multiple different healthcare facilities. In this case, the system may select a healthcare facility for the patient. Additionally, as patients are placed, the system may identify that a patient cannot be placed in remaining available healthcare facilities. In this case, the system may move a patient who had multiple matches to a different facility so the new patient can be also transferred. Thus, in addition to prioritizing care level and care level availability, the system may first place those patients who only have a single option for a healthcare facility. Next patients that now have a resulting single option, due to a first option no longer being available based upon placement of previous patients, may be placed. This may continue until all patients have been placed. In the case that a patient has multiple possible facilities, the system may simply pick a healthcare facility. This selection may be based upon some user defined factor, a default factors, timing, closest geographical location or proximity between the facilities, and/or the like.

It should be noted that this described example is merely illustrative and is intended to not be limiting. Other factors may be prioritized higher or have more weight than as described in this example. Additionally, this example is overly simplified and will likely be much more complex with some patients ending up in healthcare facilities that provide a higher level of care than the patient needs because the healthcare facility ends up being a best match against other factors.

While a single recommendation may be referred to, it should be noted that multiple recommendations may be generated, one for each patient. Multiple alternative recommendations may also be generated when more than one healthcare facility is identified that the patient could be transferred to. Additionally, the recommendation may be a single recommendation containing sub-recommendations. For example, the system may generate a single recommendation for a group of patients that are being transferred to one healthcare facility, with the recommendation including sub-recommendations for each patient being transferred to that healthcare facility. As another example, the system may generate a single recommendation for the entire group of patients that need to be transferred, even if the patients are being transferred to different healthcare facilities. The recommendation would then include sub-recommendations indicating the recommendation for an individual patient.

The event response recommendation system may prioritize patients needing transfers. Depending on the number of patients that need to be transferred, the resources that need to be utilized to facilitate the transfers may be depleted at some point during the transferring process. For example, the healthcare facility may only have access to so many transportation vehicles for transporting patients. Thus, until some of the transportation vehicles can return from transferring some patients, the healthcare facility will be unable to transfer additional patients. However, as should be readily understood, different patients may be more critical in making transfers. Thus, these patients may be prioritized for transfers. The prioritization may be identified based upon the characteristics of the patient. Thus, the recommendation may also include a timeline for transferring patients.

If a recommendation cannot be generated at 103, the system may notify a user at 105. A recommendation may not be generated when there are no satisfactory healthcare facilities that one or more patients can be transferred to. A recommendation may also not be generated if all patient characteristics cannot be fulfilled. In these cases, instead of not generating a recommendation at all, the system may generate a partial recommendation which provides recommendations for those patients that a healthcare facility can be identified to which the patient can be transferred. The system may then indicate those patients that a suitable facility was not identified. In this case, the system may identify the characteristic that is preventing the patient from being placed, thereby allowing the user to override the characteristic, manually select a healthcare facility for transfer, or otherwise address the issue. The recommendation may also include a best match or alternative options for the patient with an identification of what characteristics are not fulfilled with the best match or alternative options, thereby providing the user enough information to select a healthcare facility or otherwise make a plan for the patient.

If, on the other hand, a recommendation can be generated at 103, the event response recommendation system may perform at least one action at 104. An action may include providing the recommendation to a user. The user can then approve the recommendation or sub-recommendations included within the recommendation. The system or the user can then initiate transfers for the patients per the recommendation. Depending on the capabilities of the system or what the system is approved to perform without user input, initiating the transfers may include one or more steps, for example, initiating the orders and effectuating the transfer orders. Initiating the transfers may include generating orders for transferring the patient. This may include creating and approving the transfer paperwork, identifying and ordering the healthcare objects needed to transfer the patient, generating any doctors' orders needed for transferring the patient, making and initiating the transportation arrangements, and/or the like.

In the case that the system is fully automated, a user may not need to approve the recommendation and/or sub-recommendations before the system initiates and/or effectuates a transfer for the patient(s). Whether the system can automatically, without user approval, initiate the transfers may be based upon one or more factors. For example, the system may be programmed such that transfers can only be initiated if all patients can be transferred and all patient characteristics are fulfilled. As another example, the system may be programmed such that transfers can be initiated for patients who could be transferred and to wait for a user to provide instructions regarding the other patients. Thus, one action that may be performed by the system is to initiate a transfer for the patient(s) based upon the recommendation.

Another action that may be performed by the system is monitoring the patient during the transfer and once the patient has been accepted into the transferee healthcare facility. The system may then provide status updates regarding the patient back to the affected healthcare facility, including identifying if and/or when the patient gets discharged from the healthcare facility. The status updates may identifying a status on the condition of the patient, a location of the patient within the healthcare facility, orders or tests completed by the healthcare facility, and/or the like, thereby keeping the affected healthcare facility apprised of the status of the patient. Additionally, in the event that the affected healthcare facility moves to a state that the patient could be transferred back to the healthcare facility, the system may initiate and effectuate a transfer of the patient back to the affected healthcare facility.

While various other circuits, circuitry or components may be utilized in information handling devices, with a computer, server, client device or the like, an example device that may be used in implementing one or more embodiments includes a computing device in the form of a computer 10' as illustrated in FIG. 2. This example device may be a server used in one of the systems in a hospital network, or one of the remote computers connected to the hospital network. Components of computer 10' may include, but are not limited to, a processing unit 20', a system memory 30', and a system bus 22' that couples various system components including the system memory 30' to the processing unit 20'. Computer 10' may include or have access to a variety of computer readable media, including databases. The system memory 30' may include non-signal computer readable storage media, for example in the form of volatile and/or nonvolatile memory such as read only memory (ROM) and/or random access memory (RAM). By way of example, and not limitation, system memory 30' may also include an operating system, application programs, other program modules, and program data.

A user can interface with (for example, enter commands and information) the computer 10' through input devices 50'. A monitor or other type of device can also be connected to the system bus 22' via an interface, such as an output interface 360. The computer may include a database 40'. In addition to a monitor, computers may also include other peripheral output devices. The computer 10' may operate in a networked or distributed environment using logical connections to one or more other remote device(s) 80' such as other computers. The logical connections may include network interface(s) 70' to a network, such as a local area network (LAN), a wide area network (WAN), and/or a global computer network, but may also include other networks/buses.

Information handling device circuitry, as for example outlined in FIG. 2, may be used in client devices such as a personal desktop computer, a laptop computer, or smaller devices such as a tablet or a smart phone. In the latter cases, i.e., for a tablet computer and a smart phone, the circuitry outlined in FIG. 2 may be adapted to a system on chip type circuitry. The device, irrespective of the circuitry provided, may provide and receive data to/from another device, e.g., a server or system that coordinates with various other systems. As will be appreciated by one having ordinary skill in the art, other circuitry or additional circuitry from that outlined in the example of FIG. 2 may be employed in various electronic devices that are used in whole or in part to implement the systems, methods and products of the various embodiments described herein.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium, such as a non-signal storage device, and that are executed by a processor. A storage device may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a storage device is not a signal and "non-transitory" includes all media except signal media.

Program code embodied on a storage medium may be transmitted using any appropriate medium, including but not limited to wireless, wireline, optical fiber cable, RF, et cetera, or any suitable combination of the foregoing.

Program code for carrying out operations may be written in any combination of one or more programming languages. The program code may execute entirely on a single device, partly on a single device, as a standalone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and program products according to various example embodiments. It will be understood that the actions and functionality may be implemented at least in part by program instructions. These program instructions may be provided to a processor of a device, a special purpose information handling device, or other programmable data processing device to produce a machine, such that the instructions, which execute via a processor of the device implement the functions/acts specified.

It is worth noting that while specific blocks are used in the figures, and a particular ordering of blocks has been illustrated, these are nonlimiting examples. In certain contexts, two or more blocks may be combined, a block may be split into two or more blocks, or certain blocks may be re-ordered or re-organized as appropriate, as the explicit illustrated examples are used only for descriptive purposes and are not to be construed as limiting.

As used herein, the singular "a" and "an" may be construed as including the plural "one or more" unless clearly indicated otherwise.

## Claims

1. A method, the method comprising:
identifying, at an event response recommendation system, an event affecting a healthcare facility;
identifying, using the event response recommendation system, at least one patient within the healthcare facility needing transferred due to the event; and
generating, using the event response recommendation system, a recommendation for transferring the at least one patient.

2. The method of claim 1, further comprising initiating, using the event response recommendation system, a transfer for the at least one patient based upon the recommendation.

3. The method of claim 2, wherein the initiating is responsive to a user approving the recommendation.

4. The method of claim 2, wherein the initiating comprises generating orders for transferring the at least one patient.

5. The method of claim 1, wherein the recommendation identifies a transferee healthcare facility for transferring the at least one patient.

6. The method of claim 1, wherein the identifying the at least one patient comprises identifying at least one characteristic of the at least one patient.

7. The method of claim 6, wherein the generating the recommendation comprises matching the at least one characteristic to at least one healthcare facility based upon attributes of the at least one healthcare facility.

8. The method of claim 1, wherein the generating a recommendation comprises identifying a plurality of healthcare facilities for transferring the at least one patient and wherein the recommendation comprises one of the plurality of healthcare facilities located in closest geographical proximity to the healthcare facility.

9. The method of claim 1, wherein the generating a recommendation comprises receiving, at the event response recommendation system, a status of other healthcare facilities via a status transmission.

10. The method of claim 1, further comprising monitoring, using the event response recommendation system, the at least one patient after the at least one patient is transferred to another healthcare facility and providing notifications to the healthcare facility regarding a status of the at least one patient identified from the monitoring.

11. A system comprising:
a processor;
a memory device that stores instructions that, when executed by the processor, causes the system to carry out a method according to any one of the preceding claims.

12. A computer program product comprising:
a computer-readable storage device that stores executable code that, when executed by a processor, causes the product to:
identify, at an event response recommendation system, an event affecting a healthcare facility;
identify, using the event response recommendation system, at least one patient within the healthcare facility needing transferred due to the event; and
generate, using the event response recommendation system, a recommendation for transferring the at least one patient.
